# EUROPEAN PATENT APPLICATION

(11) **EP 3 078 385 A1**
(43) Date of publication of application: **12.10.2016**
(21) Application number: 16159162.3
(22) Date of filing: 25.05.2011
(51) Int. Cl.: A61K 38/13, A61K 36/47, A61K 9/00, A61K 9/107, A61K 31/765, A61P 27/02, A61P 27/04

(54) **CYCLOSPORIN EMULSIONS**

(30) Priority: 25.05.2010 US 347851 P
(62) Divisional of application: 11726545.4
(71) Applicant: ALLERGAN, INC., Irvine, CA 92612 (US)
(72) Inventor: Morgan, Aileen, Rancho Santa Margarita, CA California 92688 (US); Gore, Anuradha V., Irvine, CA California 92612 (US); Attar, Mayssa, Placentia, CA California 92870 (US); Pujara, Chetan P., Irvine, CA California 92620 (US)
(74) Representative: Hoffmann Eitle

(57) **Abstract**

Disclosed herein is a composition comprising cyclosporin A at a concentration between about 0.001% (w/v) and about 1.0% (w/v), a plant oil at a concentration between about 0.01% (w/v) and about 10% (w/v), and macrogol 15 hydroxystearate at a concentration between about 0.01 % (w/v) and about 10% (w/v).

## Description

Disclosed herein are emulsions comprising cyclosporin, a plant oil, macrogol 15 hydroxystearate, an emulsifier, and optionally a viscosity agent and other ingredients.

### BRIEF DESCRIPTION OF THE DRAWINGS

Figure 1 shows stability data for cyclosporin in the formulations identified as A and B at Table 1.1.
Figure 2 shows stability data for Purite® in Formulations A and B.

### DETAILED DESCRIPTION OF THE INVENTION

Disclosed herein are emulsions comprising cyclosporin A, at a concentration of from about 0.01% (w/v) to about 0.05% (w/v), and macrogol 15 hydroxystearate, as defined in claim 1. The compositions are effective to treat dry eye associated with keratoconjunctivitis sicca, to restore corneal sensitivity that has been impaired due to corneal surgery, to treat atopic and vernal keratoconjunctivitis, and to treat ptyregia, among other conditions.

### Cyclosporin A

Cyclosporin A is a cyclic peptide having the following chemical structure:

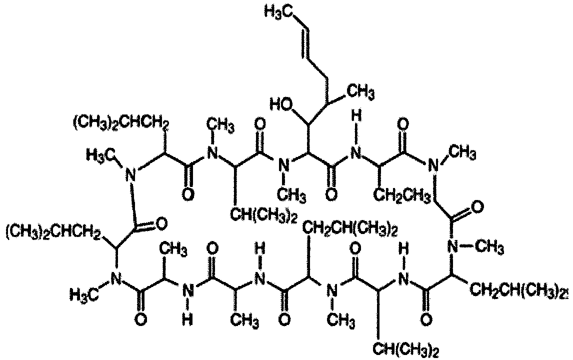

Its chemical name is Cyclo[[(*E*)-(2*S*,3*R*,4*R*)-3-hydroxy-4-methyl-2-(methylamino)-6-octenoyl]-L-2-aminobutyryl-*N*-methylglycyl-*N*-methyl-Lleucyl-L-valyl-*N*-methyl-L-leucyl-L-alanyl-D-alanyl-*N*-methyl-L-leucyl-*N*-methyl-L-leucyl-*N*methyl-L-valyl]. It is also known by the names cyclosporine, cyclosporine A, ciclosporin, and ciclosporin A.

Cyclosporin A is the active ingredient in Restasis® (Allergan, Inc., Irvine, California), an emulsion comprising 0.05% (w/v) cyclosporin. Restasis is indicated to increase tear production in patients whose tear production is presumed to be suppressed due to ocular inflammation associated with keratoconjunctivitis sicca.

Compositions of the invention comprise from about 0.01 % (w/v) to about 0.05% (w/v) cyclosporin A. As used here, the term "about" when used in connection with a value, means that the value may not differ by more than 5%. Hence, "about 1.0%" includes all values within the range of 0.95% to 1.05%.

In one embodiment, the composition comprises from about 0.01 % (w/v) to less than about 0.05% (w/v) cyclosporin A. In another embodiment, the composition comprises from about 0.01% (w/v) to about 0.04% (w/v) cyclosporin A.

In other embodiments, the compositions comprise about 0.01% (w/v), about 0.015% (w/v), about 0.02% (w/v), about 0.025% (w/v), about 0.03% (w/v), about 0.035% (w/v), about 0.04% (w/v), about 0.045% (w/v) or about 0.05% (w/v) cyclosporin A.

### Plant oils

The compositions of the invention further comprise, in addition to cyclosporin, a plant oil. The plant oil provides the oil phase of the emulsion. Suitable plant oils include, for example, anise oil, castor oil, clove oil, cassia oil, cinnamon oil; almond oil, corn oil, arachis oil, cottonseed oil, safflower oil, maize oil, linseed oil, rapeseed oil, soybean oil, olive oil, caraway oil, rosemary oil, peanut oil, peppermint oil, sunflower oil, eucalyptus oil, sesame oil, coriander oil, lavender oil, citronella oil, juniper oil, lemon oil, orange oil, clary sage oil, nutmeg oil, tea tree oil, coconut oil, tallow oil, and lard.

The composition of the invention comprises between about 0.01 % (w/v) and about 10% (w/v) of the plant oil. In another embodiment, the composition of the invention comprises between about 0.1 % (w/v) and about 1 % (w/v) of a plant oil. In another embodiment, the composition comprises about 0.01% (w/v), about 0.02% (w/v), about 0.03% (w/v), about 0.04% (w/v), about 0.05% (w/v), about 0.06% (w/v), about 0.07% (w/v), about 0.08% (w/v), about 0.09% (w/v), about 0.1 % (w/v), about 0.15% (w/v), about 0.2% (w/v), about 0.25% (w/v), about 0.3% (w/v), about 0.35% (w/v), about 0.4% (w/v), about 0.45% (w/v), about 0.5% (w/v), about 0.55% (w/v), about 0.6% (w/v), about 0.65% (w/v), about 0.7% (w/v), about 0.75% (w/v), about 0.8% (w/v), about 0.85% (w/v), about 0.9% (w/v), about 0.95% (w/v), about 1% (w/v), about 1.5% (w/v), about 2% (w/v), about 2.5% (w/v), about 3% (w/v), about 3.5% (w/v), about 4% (w/v), about 4.5% (w/v), about 5% (w/v), about 5.5% (w/v), about 6% (w/v), about 6.5% (w/v), about 7% (w/v), about 7.5% (w/v), about 8% (w/v), about 8.5% (w/v), about 9% (w/v), about 9.5% (w/v), or about 10% (w/v) of a plant oil.

### Macrogol 15 Hydroxystearate

The compositions of the invention further comprise macrogol 15 hydroxystearate, an emulsifier. Macrogol 15 hydroxystearate is a mixture of mainly monoesters and diesters of 12-hydroxystearic acid and macrogois obtained by the ethoxylation of 12-hydroxystearic acid. Macrogol 15 hydroxystearate is also known as 12-hydroxyoctadecanoic acid polymer with α-hydro-ω-hydroxypoly(oxy-1,2-ethanediyl); 12-hydroxystearic acid polyethylene glycol copolymer; macrogoli 15 hydroxystearas; polyethylene glycol-15-hydroxystearate; and polyethylene glycol 660 12-hydroxystearate.

In one embodiment, the macrogol 15 hydroxystearate is Solutol® HS 15 (BASF AG, Germany). Solutol® HS 15 consists of polyglycol mono- and di-esters of 12-hydroxystearicacid (lipophilic part), with the remaining 30% free polyethylene glycol (hydrophilic part). The main components of the lipophilic part have the following chemical structures:

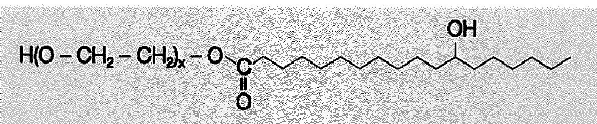

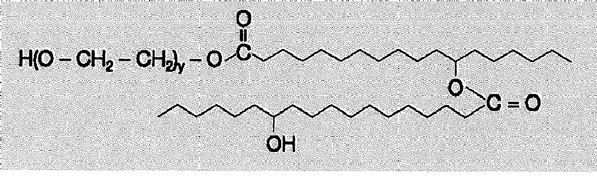

The compositions of the invention comprise macrogol 15 hydroxystearate in an amount between about 0.01% (w/v) and about 10% (w/v). In one embodiment, the composition comprises between about 0.1 % (w/v) and about 1% (w/v) macrogol 15 hydroxystearate. In one embodiment, the composition comprises between about 0.5% (w/v) and about 0.75% (w/v) macrogol 15 hydroxystearate.

In other embodiments, the compositions comprise about 0.1% (w/v), about 0.15% (w/v), about 0.2% (w/v), about 0.25% (w/v), about 0.3% (w/v), about 0.35% (w/v), about 0.4% (w/v), about 0.45% (w/v), about 0.5% (w/v), about 0.55% (w/v), about 0.6% (w/v), about 0.65% (w/v), about 0.7% (w/v), about 0.75% (w/v), about 0.8% (w/v), about 0.85% (w/v), about 0.9% (w/v), about 0.95% (w/v), or about 1 % (w/v) macrogol 15 hydroxystearate.

### Other emulsifiers

In one embodiment, the compositions of the invention further comprise, in addition to cyclosporin and macrogol 15 hydroxystearate, additional emulsifiers such as polysorbate 80 and/or POE-40 stearate. Polysorbate 80 is also known as polyoxyethylene (20) sorbitan monooleate. It has an oleate cap as shown in the structure below: 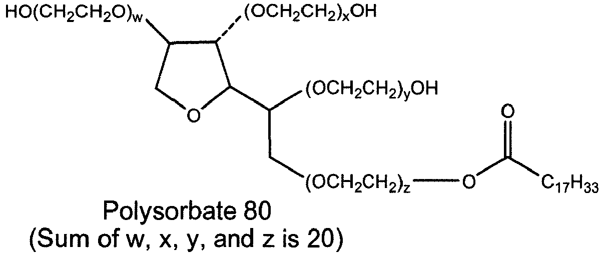 It is named POE (w+x+y+z) sorbitan mono (or di- or tri-) fatty acid, hence, polysorbate 80 is POE (20) sorbitan monooleate. POE-40 stearate is also known as 2-hydroxyethyl octadecanoate.

In another embodiment, the compositions of the invention further comprise, in addition to cyclosporin and macrogol 15 hydroxystearate, a viscosity agent or emulsifier such as Pemulen® TR-2, hydroxypropyl methyl cellulose, or carboxymethyl cellulose. Pemulen® is the trade name for high molecular weight, crosslinked copolymers of acrylic acid and C10-C30 alkyl acrylate produced by Lubrizol Corp. Pemulen® TR-2 is a C10-30 alkyl acrylate crosspolymer containing a higher level of hydrophobic groups than other Pemulen® polymers.

In one embodiment, the composition of the invention comprises between about 0.01% (w/v) and about 10% (w/v) of polysorbate 80 or POE-40 stearate, and between about 0.01 % (w/v) and about 10% (w/v) of Pemulen® TR-2, hydroxypropyl methyl cellulose, or carboxymethyl cellulose. In another embodiment, the composition of the invention comprises between about 0.01 % (w/v) and about 10% (w/v) of polysorbate 80 and POE-40 stearate, and between about 0.01% (w/v) and about 10% (w/v) of Pemulen® TR-2, hydroxypropyl methyl cellulose, and carboxymethyl cellulose.

In one embodiment, the composition of the invention comprises between about 0.01% (w/v) and about 1% (w/v) of polysorbate 80 or POE-40 stearate, and between about 0.01% (w/v) and about 1% (w/v) of Pemulen® TR-2, hydroxypropyl methyl cellulose, or carboxymethyl cellulose. In another embodiment, the composition of the invention comprises between about 0.01% (w/v) and about 1% (w/v) of polysorbate 80 and POE-40 stearate, and between about 0.01% (w/v) and about 1% (w/v) of Pemulen® TR-2, hydroxypropyl methyl cellulose, and carboxymethyl cellulose.

In another embodiment, the compositions comprise one or more of polysorbate 80, POE-40 stearate, Pemulen® TR-2, hydroxypropyl methyl cellulose, and carboxymethyl cellulose, each at one of the following concentrations: about 0.01% (w/v), about 0.02% (w/v), about 0.03% (w/v), about 0.04% (w/v), about 0.05% (w/v), about 0.06% (w/v), about 0.07% (w/v), about 0.08% (w/v), about 0.09% (w/v), about 0.1 % (w/v), about 0.15% (w/v), about 0.2% (w/v), about 0.25% (w/v), about 0.3% (w/v), about 0.35% (w/v), about 0.4% (w/v), about 0.45% (w/v), about 0.5% (w/v), about 0.55% (w/v), about 0.6% (w/v), about 0.65% (w/v), about 0.7% (w/v), about 0.75% (w/v), about 0.8% (w/v), about 0.85% (w/v), about 0.9% (w/v), about 0.95% (w/v), about 1% (w/v), about 1.5% (w/v), about 2% (w/v), about 2.5% (w/v), about 3% (w/v), about 3.5% (w/v), about 4% (w/v), about 4.5% (w/v), about 5% (w/v), about 5.5% (w/v), about 6% (w/v), about 6.5% (w/v), about 7% (w/v), about 7.5% (w/v), about 8% (w/v), about 8.5% (w/v), about 9% (w/v), about 9.5% (w/v), or about 10% (w/v).

### Additional ingredients

Tonicity agents may be added to the compositions of the invention as needed. They include, but are not limited to, salts, particularly sodium chloride, potassium chloride, mannitol and glycerin, or any other suitable ophthalmically acceptable tonicity adjustor. In one embodiment, the tonicity agent is present in an amount of between about 0.1 % (w/v) and about 10% (w/v). In another embodiment, the tonicity agent is present in an amount of between about 1.0% and 1.2%.

The vehicle for the composition is saline, water, or some other physiologically compatible vehicle.

The composition is maintained at a comfortable pH with an appropriate buffer system. A desirable pH is 7.4 - 7.6. Various buffers and means for adjusting pH may be used so long as the resulting preparation is ophthalmically acceptable. Accordingly, buffers include, but are not limited to, acetate buffers, citrate buffers, phosphate buffers and borate buffers. Acids or bases may be used to adjust the pH of these formulations as needed. In one embodiment, the buffer is boric acid at a concentration of between about 0.6% (w/v) and about 0.7% (w/v).

The composition of the invention may also include preservatives, such as Purite®, a stabilized oxychloro complex. In one embodiment, the Purite® is present at a concentration of about 0.01% (w/v).

### Examples

The inventors made the following compositions. The amount of each ingredient is listed as % (w/v).

**TABLE 1.1 - Compositions of the invention comprising Purite®.**

| **COMPONENT** | **INGREDIENT** | **A** | **B** | **C** | **D** | **E** | **F** |
|---|---|---|---|---|---|---|---|
| Active | Cyclosporine A | 0.04 | 0.04 | 0.04 | 0.04 | 0.04 | 0.04 |
| Oil Phase | Castor Oil | 0.5 | 0.5 | 0.5 | 0.5 | 0.5 | 0.5 |
| Emulsifier | Polysorbate 80 | 0.5 | -- | 0.5 | 0.25 | -- | 1.0 |
| | POE-40 Sterate | -- | 0.5 | -- | -- | 0.5 | -- |
| | Solutol-15 HS | 0.5 | 0.5 | 0.5 | 0.75 | 0.5 | 0.1 |
| Co-emulsifier/ viscosity agent | Pemulen TR-2 | 0.1 | -- | 0.075 | 0.075 | -- | -- |
| | HPMC | -- | -- | -- | -- | 0.5 | -- |
| | CMC (med. viscosity) | -- | -- | -- | -- | -- | -- |
| | CMC (low viscosity) | -- | -- | -- | -- | -- | -- |
| Tonicity agent | Glycerin | 1.0 | 1.2 | 1.2 | 1.2 | 1.2 | 1.0 |
| Buffer compoents | Boric acid | 0.6 | 0.6 | 0.7 | 0.7 | 0.7 | 0.6 |
| Preservative | Purite | 0.01 | 0.01 | 0.01 | 0.01 | 0.01 | 0.01 |
| Vehicle | Water | qs | qs | qs | qs | qs | qs |
| pH | -- | 7.4 | 7.4 | 7.4 | 7.4 | 7.4 | 7.4 |

**TABLE 1.2 - Additional compositions of the invention comprising Purite®.**

| **COMPONENT** | **INGREDIENT** | **G** | **H** | **I** | **J** | **K** | **L** | **M** |
|---|---|---|---|---|---|---|---|---|
| Active | Cyclosporine A | 0.04 | 0.04 | 0.04 | 0.04 | < 0.04 | 0.04 | < 0.04 |
| Oil Phase | Castor Oil | 0.5 | 0.5 | 0.5 | 0.5 | 0.5 | 0.5 | 0.5 |
| Emulsifier | Polysorbate 80 | 0.5 | 0.5 | 0.5 | 0.5 | 0.5 | -- | -- |
| | POE-40 Sterate | 0.5 | 0.5 | -- | -- | -- | 0.5 | 0.5 |
| | Solutol-15 HS | -- | -- | 0.5 | 1.0 | 0.5 | 0.5 | 0.5 |
| Co-emulsifier/ viscosity agent | Pemulen TR-2 | 0.5 | -- | -- | 0.1 | 0.1 | -- | -- |
| | HPMC | -- | -- | -- | -- | -- | -- | -- |
| | CMC (med. viscosity) | 0.5 | -- | -- | -- | -- | -- | -- |
| | CMC (low viscosity) | -- | 0.1 | -- | -- | -- | -- | -- |
| Tonicity agent | Glycerin | 1.0 | 1.2 | 1.0 | 1.2 | 1.2 | 1.2 | 1.2 |
| Buffer compoents | Boric acid | 0.6 | 0.7 | 0.6 | 0.6 | 0.6 | 0.6 | 0.6 |
| Preservative | Purite | 0.01 | 0.01 | 0.01 | 0.01 | 0.006-0.01 | 0.01 | 0.002 - 0.01 |
| Vehicle | Water | qs | qs | qs | qs | qs | qs | qs |
| pH | -- | 7.4 | 7.4 | | | | | |

**TABLE 2 - Compositions of the invention lacking Purite®**

| **COMPONENT** | **INGREDIENT** | **I** | **J** | **K** |
|---|---|---|---|---|
| Active | Cyclosporine A | 0.04 | 0.04 | 0.03 |
| Oil Phase | Castor Oil | 0.5 | 0.5 | 0.5 |
| Emulsifier | Polysorbate 80 | 0.5 | -- | 0.5 |
| | POE-40 Sterate | -- | 0.5 | -- |
| | Solutol-15 HS | 0.5 | 0.5 | 0.5 |
| Co-emulsifier/ viscosity agent | Pemulen TR-2 | 0.1 | -- | 0.1 |
| | HPMC | -- | -- | -- |
| | CMC (med. viscosity) | -- | -- | -- |
| | CMC (low viscosity) | -- | -- | -- |
| Tonicity agent | Glycerin | 1.2 | 1.2 | 1.2 |
| Buffer compoents | Boric acid | 0.7 | 0.7 | 0.7 |
| Preservative | Purite | -- | -- | -- |
| Vehicle | Water | qs | qs | qs |
| pH | -- | 7.4 | 7.4 | 7.4 |

### Methods of treatment

Compositions of the inventions may be used to treat patients suffering from dry eye associated with keratoconjunctivitis sicca, to restore corneal sensitivity that has been impaired due to refractive surgery on the eye (such as photorefractive keratectomy, laser assisted sub-epithelium keratomileusis (LASEK), EPI-LASEK, and customized transepithelial non-contact ablation), to treat atopic and vernal keratoconjunctivitis, and to treat ptyregia, among other conditions that are known to be amenable to treatment with topical cyclosporin at the concentrations stated here.

### EXAMPLES

The inventors tested compositions of the invention and obtained the data described below.

### Microbial Activity

Compositions A, B, and I were formulated as described in Tables 1.1 and 1.2. The compositions were sterilized by filtering through a 0.2 µm pore-size membrane filter, thereby simplifying the manufacturing process. The compositions were then tested according to the protocols established by the United States Pharmacopeia and published as the "Antimicrobial Effectiveness Test" (USPC, chapter 51). In accordance with this test, the compositions of the invention were tested by adding four microorganisms *(S. aureus, P. aeruginosa, C. albicans, and A. brasiliensis*) directly to the compositions at relatively high concentrations to simulate contamination. The compositions were held for 28 days, with analysis of microorganism levels at 6 hours, 24 hours, and 7, 14, and 28 days. The test was performed twice with each compsition.

As Tables 3.1 and 3.2, below, show, compositions A, B, and I had unexpectedly high antimicrobial preservative efficacy.

**TABLE 3.1 - Results of Antimicrobial Effectiveness Test, test 1. Sa = S. auereus, Pa. = P. aeruginosa, Ca = C. albicans, Ab = A. brasiliensis. Values shown are logarithmic drops in microbial levels.**

| **SAMPLE** | **6 HR.** | 24 **HR.** | 7 **DAYS** | 14 **DAYS** | **28 DAYS** |
|---|---|---|---|---|---|
| A | Sa. 3.8 | Sa. >4.8 | Sa. >4.8 | Sa. >4.8 | Sa. >4.8 |
| | Pa. >4.7 | Pa. >4.7 | Pa. >4.7 | Pa. >4.7 | Pa. >4.7 |
| | | | Ca. >4.7 | Ca. >4.7 | Ca. >4.7 |
| | | | Ab. >4.2 | Ab. >4.2 | Ab. >4.2 |
| I | Sa. 3.3L | Sa. >4.8 | Sa. >4.8 | Sa. >4.8 | Sa. >4.8 |
| | Pa. >4.7 | Pa. >4.7 | Pa. >4.7 | Pa. >4.7 | Pa. >4.7 |
| | | | Ca. >4.7 | Ca. >4.7 | Ca. >4.7 |
| | | | Ab. >4.2 | Ab. >4.2 | Ab. >4.2 |
| B | Sa. >4.7 | Sa. >4.7 | Sa. >4.7 | Sa. >4.7 | Sa. >4.7 |
| | Pa. >4.2 | Pa. >4.2 | Pa. >4.2 | Pa. >4.2 | Pa. >4.2 |
| | | | Ca. >4.6 | Ca. >4.6 | Ca. >4.6 |
| | | | Ab. >2.7 | Ab. >3.7 | Ab. >3.7 |

**Table 3.2 - Results of Antimicrobial Effectiveness Test, test 2. Sa = S. auereus, Pa. = P. aeruginosa, Ca = C. albicans, Ab = A. brasiliensis. Values shown are logarithmic drops in microbial levels.**

| **SAMPLE** | **6 HR.** | **24 HR.** | **7 DAYS** | **14 DAYS** | **28 DAYS** |
|---|---|---|---|---|---|
| A | Sa. 3.7 | Sa. >4.7 | Sa. >4.7 | Sa. >4.7 | Sa. >4.7 |
| | Pa. >4.7 | Pa. >4.7 | Pa. >4.7 | Pa. >4.7 | Pa. >4.7 |
| | | | Ca. >4.6 | Ca. >4.6 | Ca. >4.6 |
| | | | Ab. >4.1 | Ab. >4.1 | Ab. >4.1 |
| I | Sa. 3.5 | Sa. >4.7 | Sa. >4.7 | Sa. >4.7 | Sa. >4.7 |
| | Pa. >4.7 | Pa. >4.7 | Pa. >4.7 | Pa. >4.7 | Pa. >4.7 |
| | | | Ca. >4.6 | Ca. >4.6 | Ca. >4.6 |
| | | | Ab. >4.1 | Ab. >4.1 | Ab. >4.1 |
| B | Sa. 3.7 | Sa. 3.7 | Sa. >4.7 | Sa. >4.7 | Sa. >4.7 |
| | Pa. >4.2 | Pa. >4.2 | Pa. >4.2 | Pa. >4.2 | Pa. >4.2 |
| | | | Ca. >4.6 | Ca. >4.6 | Ca. >4.6 |
| | | | Ab. >2.6 | Ab. >3.7 | Ab. >3.7 |

### Pharmacokinetics

The inventors sought to assess the pharmacokinetics and distribution in ocular tissues of Restasis® and compositions A and B following a single bilateral topical ocular administration to New Zealand White [Hra:(NZW)SPF] rabbits. Restasis® and compositions A and B were administered to 42 rabbits, such that 14 rabbits received one of those compositions. Restasis® was dosed at 17.5 µg per eye; compositions were dosed at 14 µg per eye. Each animal received 35 µL of the dose formulation in each eye. The dose was administered into the cul-de-sac of the eye via a positive displacement micropipette, ensuring contact with the conjunctiva. After the dose was administered, the upper and lower eyelids were gently held together for approximately 5 seconds to prevent the loss of material and distribute the dose across the eye. Each animal was restrained for approximately 1 minute to prevent rubbing of the eyes. If excess dose formulation flowed out of the eye, the lower lid was blotted with a gauze pad (dose wipe). Environmental controls for the animal room were set to maintain a temperature of 16 to 22 °C, a relative humidity of 50±20%, and a 12-hour light/12-hour dark cycle. The 12-hour dark cycle was interrupted to accommodate study procedures.

Animals were anesthetized with sodium pentobarbital (using a portion of a 1 mL/kg, 65 mg/mL solution) and blood was collected from 2 animals/group/time point via cardiac puncture at 0.5, 2, 6, 12, 24, 48, and 144 hours post dose. For post dose intervals that include blood and tear collections, tear collections were performed immediately prior to blood collections. Blood (approximately 5 mL) was collected into a tube containing K₃EDTA and immediately transferred into silanized tubes with screw tops. Following blood collection, animals were sacrificed, both eyes enucleated and thoroughly rinsed with 0.9% saline. Ocular tissues were then collected as single samples. Ocular tissues were analyzed for cyclosporin-A using liquid chromatography with tandem mass spectrometry (LC-MS/MS) analysis.

The results are presented in Table 4, below.

**TABLE 4 - Cyclosporin A concentrations in certain ocular tissues of formulations A and B compared to Restasis®**

| **SAMPLE** | **CORNEA** | | | **BULBAR CONJUNCTIVA** | | | **PALPEBRAL CONJUNCTIVA** | | |
|---|---|---|---|---|---|---|---|---|---|
| RESTASIS® | | | | | | | | | |
| Mean ±SD/SE | 272 ±212 | 13600 ±2300 | 100 | 141 ±7 | 1110 ±50 | 100 | 359 ±102 | 2940 ±230 | 100 |
| Median | 264 | 13100 | 100 | 204 | 1150 | 100 | 356 | 2890 | 100 |
| A | | | | | | | | | |
| Mean ±SD/SE | 477 ±195 | 29800 ±3100 | 219 | 296 ±84 | 2080 ±170 | 188 | 1010 ±170 | 5730 ±320 | 195 |
| Median | 440 | 28500 | 218 | 299 | 2150 | 187 | 1050 | 6080 | 210 |
| B | | | | | | | | | |
| Mean ±SD/SE | 664 ±301 | 13300 ±1400 | 97 | 459 ±285 | 1850 ±200 | 167 | 1660 ±270 | 4540 ±360 | 154 |
| Median | 667 | 14100 | 108 | 462 | 1950 | 170 | 1650 | 4830 | 167 |

### Stability

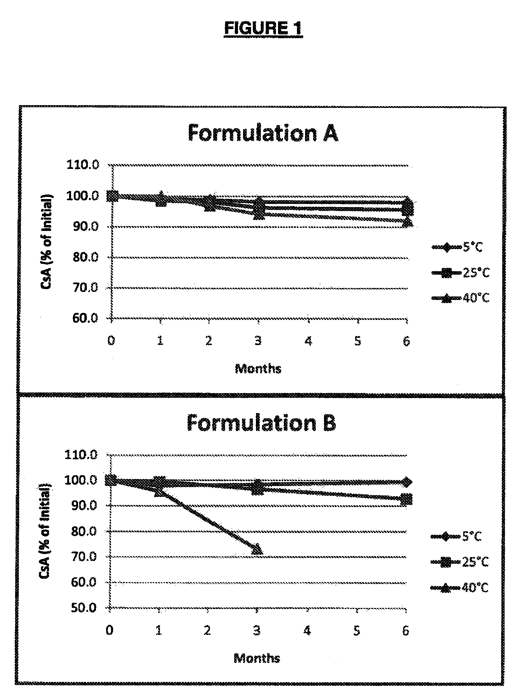

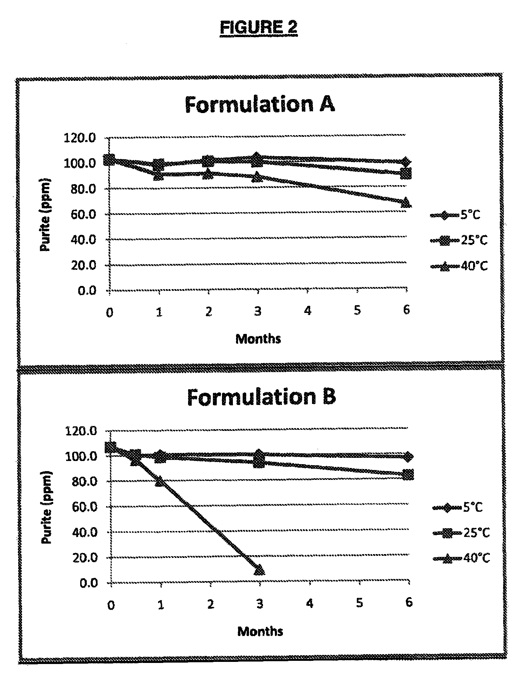

The concentration of cyclosporin A and Purite® in samples of Compositions A and B was measured over a period of six months. Concentrations of cyclosporin A was measured using HPLC, and concentrations of Purite® was measured by titration. The results are shown in Figures 1 and 2: Figure 1 shows concentrations of cyclosporin in Formulations A and B; Figure 2 shows concentrations of Purite® in Formulations A and B. The storage conditions are as shown in the figures.

The present invention includes the following items 1 to 25:
Item 1. A composition comprising the following:
   cyclosporin A at a concentration between about 0.001% (w/v) and about 1.0% (w/v);
   a plant oil at a concentration between about 0.01% (w/v) and about 10% (w/v);
   macrogol 15 hydroxystearate at a concentration between about 0.01% (w/v) and about 10% (w/v); and
      water.
Item 2. The composition of item 1, wherein the cyclosporin is present at a concentration of between about 0.01% and about 0.05% (w/v).
Item 3. The composition of item 1, wherein the macrogol 15 hydroxystearate is Solutol® HS 15 and is present at a concentration of between about 0.25% (w/v) and about 0.75% (w/v).
Item 4. The composition of item 3, wherein the plant oil is anise oil, castor oil, clove oil, cassia oil, cinnamon oil; almond oil, corn oil, arachis oil, cottonseed oil, safflower oil, maize oil, linseed oil, rapeseed oil, soybean oil, olive oil, caraway oil, rosemary oil, peanut oil, peppermint oil, sunflower oil, eucalyptus oil, sesame oil, coriander oil, lavender oil, citronella oil, juniper oil, lemon oil, orange oil, clary sage oil, nutmeg oil, tea tree oil, coconut oil, tallow oil, or lard.
Item 5. The composition of item 4, wherein the composition comprises castor oil at a concentration of between about 0.25% (w/v) and about 0.5%.
Item 6. The composition of any of item 5, further comprising a tonicity agent at a concentration between about 0.1 % (w/v) and about 10% (w/v).
Item 7. The composition of item 6, wherein the tonicity agent is glycerin, sodium chloride, potassium chloride, or mannitol.
Item 8. The composition of item 7, wherein the tonicity agent is glycerin at a concentration of between about 1.0% (w/v) and about 1.5% (w/v).
Item 9. The composition of item 8, further comprising a buffer.
Item 10. The composition of item 9, wherein the buffer is an acetate buffer, a citrate buffer, a phosphate buffers, or a borate buffer.
Item 11. The composition of item 10, wherein the buffer is boric acid at a concentration of between about 0.6% (w/v) and about 0.7% (w/v).
Item 12. The composition of any of item 11, further comprising Polysorbate 80 at a concentration of between about 0.1 % (w/v) and about 10% (w/v).
Item 13. The composition of item 12, wherein the Polysorbate 80 is present at a concentration of between about 0.25% and about 0.5% (w/v).
Item 14. The composition of item 13, further comprising POE-40 stearate at a concentration of between about 0.1 % and about 10% (w/v).
Item 15. The composition of item 14, wherein the POE-40 stearate is present at a concentration of about 0.5% (w/v).
Item 16. The composition of item 14, further comprising Pemulen Tr-2 at a concentration of between about 0.01 % (w/v) and about 10% (w/v).
Item 17. The composition of item 16, wherein the Pemulen Tr-2 is present at a concentration of between about 0.075% (w/v) and about 0.1 % (w/v).
Item 18. The composition of item 17, further comprising hydroxypropyl methyl cellulose or carboxymethyl cellulose at a concentration of between about 0.01% (w/v) and about 10% (w/v).
Item 19. The composition of item 18, wherein the hydroxypropyl methyl cellulose or carboxymethyl cellulose is present at a concentration of between about 0.1 % (w/v) and about 0.5% (w/v).
Item 20. The composition of item 16, further comprising Purite at a concentration of about 0.001 % (w/v) to about 1 % (w/v).
Item 21. The composition of item 20, wherein the Purite is present at a concentration of about 0.01% (w/v).
Item 22. A method of treating keratoconjunctivitis sicca, the method comprising administering to the eye of a mammal a composition according to item 1.
Item 23. A method of restoring corneal sensitivity in a mammal after refractive surgery on the eye, the method comprising administering to the eye of a mammal a composition according to item 1.
Item 24. A method of treating atopic or vernal keratoconjunctivitis, the method comprising administering to the eye of a mammal a composition according to item 1.
Item 25. A method of treating pterygia, the method comprising administering to the eye of a mammal a composition according to item 1.

## Claims

1. A composition for use in a method for the treatment of atopic or vernal keratoconjunctivitis, the method comprising administering to the eye of a mammal said composition comprising:
cyclosporin A at a concentration between about 0.001% (w/v) and about 1.0% (w/v);
a plant oil at a concentration between about 0.01% (w/v) and about 10% (w/v);
macrogol 15 hydroxystearate at a concentration between about 0.01% (w/v) and about 10% (w/v); and
water.

2. The composition of claim 1, wherein the cyclosporin is present at a concentration of between about 0.01% and about 0.05% (w/v).

3. The composition of claim 1, wherein the macrogol 15 hydroxystearate is Solutol® HS 15 and is present at a concentration of between about 0.25% (w/v) and about 0.75% (w/v).

4. The composition of any of claims 1-3, wherein the plant oil is anise oil, castor oil, clove oil, cassia oil, cinnamon oil; almond oil, corn oil, arachis oil, cottonseed oil, safflower oil, maize oil, linseed oil, rapeseed oil, soybean oil, olive oil, caraway oil, rosemary oil, peanut oil, peppermint oil, sunflower oil, eucalyptus oil, sesame oil, coriander oil, lavender oil, citronella oil, juniper oil, lemon oil, orange oil, clary sage oil, nutmeg oil, tea tree oil, coconut oil, tallow oil, or lard, and preferably is castor oil at a concentration between about 0.25% (w/v) and about 0.5% (w/v).

5. The composition of any of claims 1-4, further comprising a tonicity agent at a concentration between about 0.1 % (w/v) and about 10% (w/v),
wherein the tonicity agent is preferably glycerin, sodium chloride, potassium chloride, or mannitol, and more preferably is glycerin at a concentration of between about 1.0% (w/v) and about 1.5% (w/v).

6. The composition of any of claims 1-5, further comprising a buffer,
wherein the buffer is preferably an acetate buffer, a citrate buffer, a phosphate buffer, or a borate buffer,
wherein the buffer more preferably is boric acid at a concentration of between about 0.6% (w/v) and about about 0.7% (w/v).

7. The composition of any of claims 1-6, further comprising Polysorbate 80 at a concentration of between about 0.1 % (w/v) and about 10% (w/v), preferably between about 0.25% (w/v) and about 0.5% (w/v).

8. The composition of any of claims 1-7, further comprising POE-40 stearate at a concentration of between about 0.1 % and about 10% (w/v).

9. The composition of any of claims 1-8, wherein the POE-40 stearate is present at a concentration of about 0.5% (w/v).

10. The composition of any of claims 1-9, further comprising Pemulen Tr-2 at a concentration of between about 0.01% (w/v) and about 10% (w/v).

11. The composition of any of claims 1-10, wherein the Pemulen Tr-2 is present at a concentration of between about 0.075% (w/v) and about 0.1% (w/v).

12. The composition of any of claims 1-11, further comprising hydroxypropyl methyl cellulose or carboxymethyl cellulose at a concentration of between about 0.01% (w/v) and about 10% (w/v).

13. The composition of any of claims 1-12, wherein the hydroxypropyl methyl cellulose or carboxymethyl cellulose is present at a concentration of between about 0.1% (w/v) and about 0.5% (w/v).

14. The composition of any of claims 1-13, further comprising Purite at a concentration of about 0.001 % (w/v) to about 1 % (w/v).

15. The composition of any of claims 1-14, wherein the Purite is present at a concentration of about 0.01 % (w/v).
